(19) Europäisches Patentamt
European Patent Office
Office européen des brevets

(11) Publication number: 0 410 380 A2

# (12) EUROPEAN PATENT APPLICATION

(21) Application number: **90114161.4**

(22) Date of filing: **24.07.90**

(51) Int. Cl.5: **C07C 17/10**, C07C 22/04, C07C 22/00, C07D 333/12

(30) Priority: **25.07.89 US 384943**

(43) Date of publication of application: **30.01.91 Bulletin 91/05**

(84) Designated Contracting States: **AT BE CH DE DK ES FR GB GR IT LI LU NL SE**

(71) Applicant: **MERRELL DOW PHARMACEUTICALS INC.**
**2110 East Galbraith Road**
**Cincinnati Ohio 45215-6300(US)**

(72) Inventor: **McCarthy, James R.**
**6448 Foxview Place**
**West Chester, Ohio 45069(US)**
Inventor: **Matthews, Donald P.**
**7911 Red Mill Drive**
**West Chester, Ohio 45069(US)**
Inventor: **Barney, Charlotte L.**
**6152 Fairway Drive**
**Cincinnati, Ohio 45212(US)**

(74) Representative: **Vossius & Partnerner**
**Siebertstrasse 4 P.O. Box 86 07 67**
**D-8000 München 86(DE)**

## (54) Novel process for the synthesis of 2-fluoro-1-olefins.

(57) A process for preparing 2-fluoro-1-olefins or terminal bis-beta-fluoro-olefins which comprises reacting an appropriate allyl or alpha-olefinated starting material with phenylselenyl chloride and silver fluoride, and then reacting the intermediate phenylselenyl fluoride compound with ozone, and further reacting the resulting phenylselenoxide compound with an appropriate amine, to yield the desired fluorinated compounds.

EP 0 410 380 A2

# NOVEL PROCESS FOR THE SYNTHESIS OF 2-FLUORO 1-OLEFINS

Synthetic procedures for the preparation of fluorinated compounds are of interest due to the importance of these compounds as pharmaceutical agents and as probes for understanding various metabolic and enzymatic processes. For example, P. Bey, et al , have disclosed 2-(3,4-dimethoxyphenyl)-3-fluoroallylamine, which is active as a monoamine oxidase inhibitor, in J. Med. Chem ., 27 , 9 (1984), and M. Kolb, et al , have reported on monofluoroethenyl-GABA derivatives, which are active as GABA-transaminase inhibitors, in J. Med. Chem ., 30 , 267 (1987). 2-Fluoro-1-olefin compounds have been found to be useful as pharmacological agents (see European Patent Application filed concurrently herewith , i.e. on July 24, 1990,and entitled PHARMACEUTICALLY ACTIVE 3-ARYL AND 3-HETEROARYL-2-FLUORO-1-OLEFINS). Therefore, a need exists for a facile process by which to synthesize 2-fluoro-1-olefin compounds.

Bromination of vinylic compounds, where a phenylselenidyl intermediate is oxidized by means of ozone, and further reacted with diisopropylamine in the presence of heat to produce the desired 2-bromo-1-olefin, is disclosed in Tetrahedron Letters No. 44, pp 3909-3912 (1977). Chlorination of allylic alcohols utilizing a similar procedure is disclosed in J. Org. Chem . 47 1258-1267 (1982). However, Nash and Gammill report, in Tetrahedron Letters , 28 , 4003 (1987), that the olefin-fluorination of certain olefin containing compounds through the oxidation of a fluoro-phenylselenyl intermediate is very unfavorable for steric reasons, and that the presence of fluorine would be expected to exert an unfavorable electronic influence on a final elimination step, thereby thwarting efforts to produce desired 2-fluoro-olefin compounds. Apparently a successful process for the preparation of 2-fluoro-1-olefin compounds through the utilization of phenylselenyl methodology heretofore did not exist.

This invention relates to the process for preparing 2-fluoro-1-olefin and terminal bis-beta-fluoro-olefin compounds by means of reacting the appropriate allyl or alpha-olefinated starting material with phenylselenyl chloride or phenylselenyl bromide and silver fluoride to produce a fluorinated phenylselenide compound which is then reacted first with ozone and then with an appropriate amine in the presence of heat, to produce the desired 2-fluoro-1-olefin or terminal bis-beta-flouro-olefin compound.

More specifically, this invention relates to the process for preparing compounds of the formula

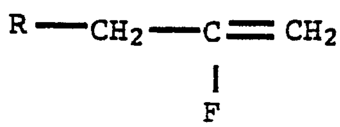

(I)

or

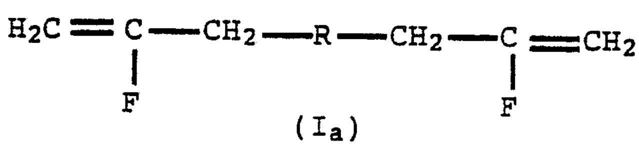

(Ia)

wherein R is a monovalent or bivalent cyclic group selected from the group consisting of optionally substituted ($C_3$-$C_{12}$)monocyclic hydrocarbons; optionally substituted 5 and 6 membered aromatic heterocycles; ($C_3$-$C_{10}$)heterocycles; and fused and bridged polycyclic groups; or

R is an optionally substituted ($C_1$-$C_{16}$)alkyl; an optionally substituted ($C_1$-$C_{16}$)heteroalkyl; or a combination of a cyclic group and an alkyl or heteroalkyl group as described above; and

wherein the optional substituents are 1 or more substituents selected from the group consisting of $C_1$-$C_{16}$)-alkyl, ($C_1$-$C_{16}$)alkoxy, hydroxy, amine, protected sulfur and nitrogen derivatives, ketone, acetal, ketal, fluoro, chloro, and bromo which comprises:

a) forming a compound of the formula III

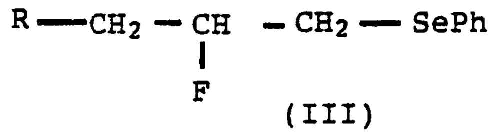

(III)

or

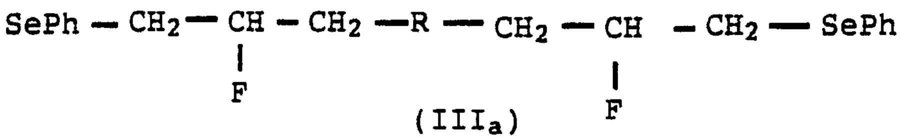

(IIIa)

wherein SePh is phenylselenide, by reacting phenylselenyl chloride or phenylselenyl bromide and silver fluoride with a compound of the formula II

$X\text{--}CH_2\text{-}CH=CH_2$ (II)

or

$H_2C=CH\text{-}CH_2\text{-}X\text{-}CH_2\text{-}CH=CH_2$ (IIa)

wherein X is R or a protecting group of R, in an aprotic solvent;

b) treating the compound of formula III with ozone in a chlorinated solvent; and

c) further reacting the phenyl selenoxide compound resulting from step b) with a non-nucleophilic amine in the presence of heat.

This process is useful for the synthesis of, for example, 2-fluoro-1-olefin compounds such as 3-(4-hydroxyphenyl)-2-fluoro-1-propene and 3-(4-methoxyphenyl)-2-fluoro-1-propene, which are useful in the treatment of hypertension.

As used herein, the term "($C_3$-$C_{12}$)monocyclic hydrocarbon" means a saturated or aromatic hydrocarbon ring containing from 3 to 12 carbon atoms and includes such groups as cyclopropyl, cyclohexyl, cycloheptyl, phenyl, phenylene, and the like. The optionally substituted ring may be attached to the propene portion of the compound at any available carbon atom to make 2-fluoro-1-propene compounds, and can be attached to each branch of the bis compounds at any available carbon atom.

The term "5 and 6 membered aromatic heterocycles" means a single aromatic cyclic group containing one or more heteroatoms selected from the group consisting of nitrogen and sulfur. Such groups include, for example, thiophene, thiazole, pyridine, imidazole, pyrimidine, and the like. The optionally substituted ring may be attached to the propene portion of the compound at any available carbon atom to make 2-fluoro-1-propene compounds, and can be attached to each branch of the bis compounds at any available carbon atom.

The term "($C_3$-$C_{10}$)heterocycles" means a single saturated ring containing one or more hetero atoms chosen from the group consisting of oxygen, sulfur and nitrogen. When a hetero atom is nitrogen, it must be present in a protected form, for instance as an amide, and when a hetero atom is sulfur, it must be present in an oxidized form, such as a sulfone, in order to avoid side reactions with ozone during the ozonolysis step of this invention. The hetero atom(s) can be located at any feasible location in the ring, and when multiple heteroatoms are present in the ring, they can be combined in any feasible manner, and can be located in any feasible position in the ring. Examples of such heterocyclic groups include thiene, tetrahydrofuran, N-acetyltetrahydropyrole, cyclopentylsulfone, dioxane, N-acetylmorpholine, and the like. The optionally substituted ring may be attached to the propene portion of the compound at any feasible and available carbon atom to make 2-fluoro-1-propene compounds, and can be attached to each branch of the bis compounds at any feasible atom.

The term "fused polycyclic groups" means 2 to 5 rings chosen from ($C_3$-$C_{12}$)monocyclic hydrocarbon, 5 and 6 membered aromatic hetrocycles, and ($C_3$-$C_{10}$)heterocycles, which feasibly can be fused, and include such groups as naphthyl, phenanthryl, 5,6,7,8-tetrahydro-2-naphthyl, 1(4H)-naphthlidenyl, 2,7-phenanthrenediyl, and the like. The optionally substituted rings may be attached to the propene portion of the compound at any feasible atom to make 2-fluoro-1-propene compounds, and can be attached to each branch of the bis compounds at any feasible atom.

The term "bridged polycyclic groups" means 2 to 5 cyclic systems chosen from the $(C_3-C_{12})$-monocyclic hydrocarbons, 5 and 6 membered aromatic heterocycles, and $(C_3-C_{10})$heterocycles described above, which are directly joined to each other by single bonds, and include such groups as biphenylyl, 4-cyclooctyl-4′-cyclopentylbiphenyl, 2-phenylnaphthalenyl, 1,2′-binaphthyl, cyclohexylbenzyl, terphenyl, binaphthalene, and the like. This term also includes cyclic systems that are joined by alkyl and heteroalkyl bridging groups, as those terms are defined below, and include such compounds as benzylnaphthalenyl, 1,2,4-tris(3-p-tolylpropyl) benzyl, benzylphenyl, methylenedioxydibenzyl, phenoxyethylbenzyl, ethylenedioxydicyclohexyl, and the like. The optionally substituted rings may be attached to the propene portion of the compound at any feasible atom to make 2-fluoro-1-propene compounds, and can be attached to any feasible atom on each branch of the bis compounds.

The term "$(C_1-C_{16})$alkyl" and "$(C_1-C_{16})$heteroalkyl" mean saturated, straight or branched chain alkyl or heteroalkyl groups having from one to 16 carbon atoms. The hetero atoms in the heteroalkyl groups can be sulfur, nitrogen or oxygen, and can be present singly or multiply in any feasible combination with each other, and at any feasible position in the alkyl chain. When a hetero atom is nitrogen, it must be present in a protected form, for instance as an amide, and when a hetero atom is sulfur, it must be present in an oxidized form, such as a sulfone, in order to avoid side reaction with ozone during the ozonolysis step of this invention. Examples of "$(C_1-C_{16})$alkyl" and "$(C_1-C_{16})$ heteroalkyl" include such groups as methyl, ethyl, n-propyl, isopropyl, n-butyl, isobutyl, sec-butyl, tert-butyl, methoxy, and the like, as well as 2,5,8,11-tetraoxatridecane, 4-oxa-13-thia-8,9,diaza-1,6,8,10,15-hexadecapentyl, succinimidoyl, and the like.

Additionally, cyclic systems such as those described above can be joined to alkyl and heteroalkyl chains such as those described above to make such groups as 2-naphthylacetyl, cyclohexanecarbohydroximoyl, thienylpropyl, phenylbutyl, phenoxypropyl, benzyloxy, phenyloxy, and the like, and these groups are contemplated as being within the scope of compounds that can be made by the process of this invention.

The term "optionally substituted" means that moieties both with and without substitutents from each group described above are contemplated and included within the scope of compounds which can be made by the process of this invention. The optional substituents may be attached to each group described above singly or multiply, in any feasible combination and at any feasible position(s) on the group. Optional substitutents include $(C_1-C_{16})$alkyl and $(C_1-C_{16})$alkoxy groups, as described in the description for alkyl and heteroalkys groups above, protected nitrogen and sulfur derivatives, such as sulfone, amide, urea, nitrile, sulfuonamide, ether, and the like, and also include ketone, ketal, fluoro, chloro and bromo groups. Examples of substituted R groups include 2-methyl-3-chlorothiophene, 3-methoxy-2-pyridinone, and the like.

The process of this invention is NOT appropriate for producing fluoroinated compounds wherein R would contain an unsaturated moiety. Unsaturated cyclic or chain-type groups which would be reactive with ozone during the ozonolysis step of this process, and therefore which would be in appropriate for purposes of this invention, include furan, cyclohexene, 1,3-cyclohexadiene, 1-cyclodecen-4-yne, 2-cyclopenten-1-yl, 2,4-cyclopentadien-1-yl, cyclopentylidene, 2,4-cyclohexadien-1-ylidene, 2,5-cyclohexadien-1,4-ylene, 3-cyclohexen-1,2-ylene, 1,3-cyclopentylene, hexaceneyl, indenyl, pyrenyl, stilbenyl, dithiobenzyl, and the like.

The process of this invention is depicted generally in Reaction Scheme 1 and Reaction Scheme 2 below.

## Reaction Scheme 1

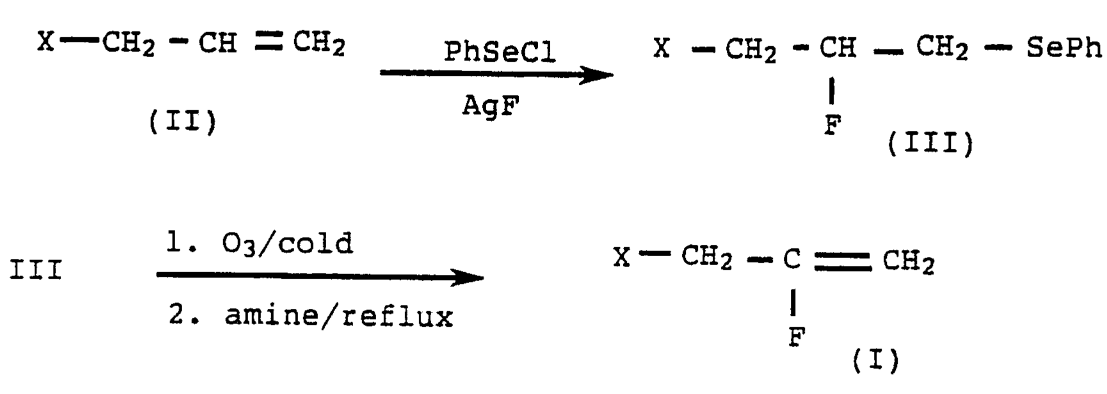


(II) (III)

III (I)

where X is R or a protecting group of R, PhSeCl is phenylselenyl chloride, AgF is silver fluoride, $O_3$ is ozone, and SePh is phenylselenide.

Reaction Scheme 2

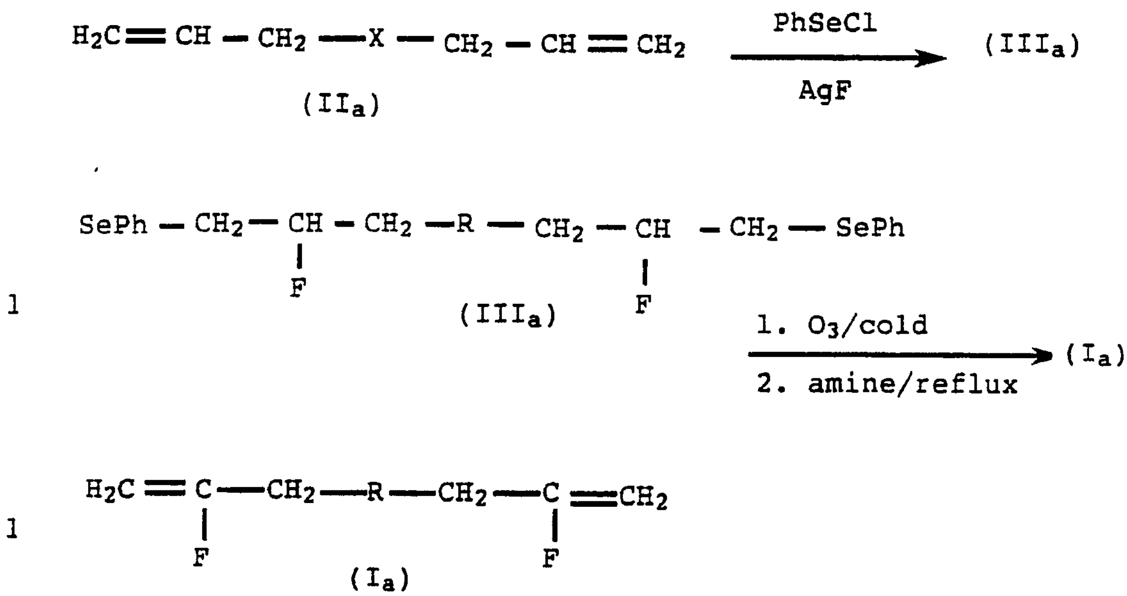


(IIa) (IIIa) (Ia)

where X is R or a protecting group of R, PhSeCl is phenylselenyl chloride, AgF is silver fluoride, $O_3$ is ozone, and SePh is phenylselenide.

Generally, the process of this invention can be described in the following manner: an appropriate compound (II or IIa) is reacted with phenylselenyl chloride (PhSeCl) or phenylselenylbromide and silver fluoride (AgF) in any aprotic solvent, preferably acetonitrile, to produce compound III or IIIa, which is then first treated, at about 25° C (i.e., room temperature) to about -25° C, preferably at -15° C to -25° C, with ozone ($O_3$) in any chlorinated solvent such as, for example, methylenechloride, hexachloroethane, or, preferably, carbon tetrachloride. Treatment with ozone will, preferably, continue until the solution is saturated with ozone. Next, an appropriate amine, preferably diisopropylamine, is added to the solution and the solution is heated to at least about 80° C, preferably at reflux, for about one to about 48 hours, preferably for about 16 hours, to produce the compounds of formula I or Ia.

Generally, silver fluoride is used in amounts equivalent to or in excess of the starting olefin in this process, and phenylselenenyl chloride or bromide is used in amounts equivalent to or in excess of the amount of starting material (i.e., a compound of formula II or IIa), although greater or lesser amounts of either silver fluoride or phenylselenenyl chloride or bromide would not prevent the process from working.

When this process is utilized for producing difluorinated compounds that have a terminal olefin on each end of the molecule, that is, bis terminal olefins, the same general procedure described above is followed, as depicted in Reaction Scheme 2, except that twice as much phenylselenyl chloride (or bromide), silver floride, and amine must be utilized in the reaction as is required to make a single, mono-fluorinated terminal olefin.

When converting the compound of formula II or IIa to the corresponding phenylselenide compound of formula III or IIIa, the term "any aprotic solvent" means any solvent which neither donates nor accepts protons, and includes such solvents as dimethylformamide, propionitrile, acetonitrile, and the like. When converting a compound of formula III or IIIa to the corresponding end product of formula I or Ia, the term "any chlorinated solvent" means any solvent that does not react with ozone, and includes such solvents as methylene chloride, carbon tetrachloride, hexachloroethane, and the like. When further converting the phenylselenoxide compound, which is produced by ozonalysis of the compound of formula III or IIIa to the corresponding end product of formula I or Ia, the term "non-nucleophilic" means any feasible non-nucleophilic amine, and includes such amines as triethylamine, diisopropylamine, diazabicycloundecene (DBU), and the like.

When a compound wherein R is an alcohol, an amine, phenol, phenyl substituted with an alcohol or amine, or the like, is the desired end product, it is necessary to start with a protecting group (X) such as benzyl, benzoyl, benzyloxy, 4-methoxybenzyloxy, methoxy, tetrahydropyran, 2-(trimethylsilyl)ethoxymethyl, or the like, as the X component in the compound of formula II or IIa, in order to avoid side reactions such as oxidations. The reaction of Scheme 1 or 2 can be followed from the beginning until the phenylselenoxide compound produced by ozonalysis of a compound of formula III or IIIa has been further treated with an

appropriate amine at reflux. Then the protecting group is removed with an appropriate deprotecting agent such as saturated methanolic hydrochloric acid, according to procedures well known in the art, to leave the desired 2-fluoro-1-propene.

This method of fluorinating these compounds is generally applicable to all terminal olefins except those terminal olefins which contain unprotected electron withdrawing groups such as nitriles, sulfones, esters, nitro groups, and the like, or which contain bulky groups such as t-butyl, neopentyl and the like.

Illustrative examples of terminal olefins, i.e., compounds of formula II, which can be fluorinated by this method include:

1-allylnaphthalene
2-allylnaphthlene
2-allylthiophene
3-allylthiophene
vinylcyclododecane
2-allyltetrahydrofuran
2-allylthiazole
3-phenyl-1-butene
3-(2-thienyl)-1-butene
allylbenzene
1,9-decadiene
3,3-dimethyl-1-butene
1-dodecene
1-eicosene
1-heptene
1-hexadecene
1-hexene
3-methyl-1-hexene
3-methyl-1-pentene
4-methyl-1-pentene
1-nonene
1-octadecene
1-octene
1-pentadecene
1-tetradecene
1-tridecene
vinylcyclohexane
vinylcyclopentane.

The above compounds, which correspond to the compounds of formula II or IIa are readily available.

The following specific examples are presented to illustrate this invention, but they should not be construed as limiting the scope of this invention in any way.

## EXAMPLE 1

### Process for Preparing 3-(4-Methoxyphenyl)-2-Fluoro-1-(Phenylselenyl)Propane (III)

To a dry 250 ml flask equipped with septum and argon bubbler was added finely ground silver fluoride (glove bag), dry acetonitrile (100 ml), phenylselenenyl chloride (4.6 g, 24 mmole) and 4-allylanisole (II) (2.96 g, 20 mmole), dissolved in 25 ml acetonitrile, was added via syringe. The reaction was stirred at room temperature for 18 hours, after which time the reaction was filtered through a celite pad and treated with about 15 ml flash silica gel. The mixture was evaporated to a powder at less than 30°C, and the title compound was separated out by flash chromatography (10% ether in hexane). The product (3.39 g, 53%) was obtained as white crystals.

M.p. 20-23°C. IH NMR (300 MHz, $CDCl_3$) δ 2.90-3.19 (m, 4), 3.79 (s, 3), 4.80 (dm, 1, J = 47.6 Hz), 6.83 (d, 2, J = 8.7 Hz), 7.10 (d, 2, J = 8.7 Hz), 7.25 (m, 3), 7.50 (m, 2). 19F NMR ($CDCl_3$) δ(vs. $CFCl_3$, 282 $MH_2$) -171.6 (dtt, J = 46.2, 27, 21 Hz): MS ($CI/CH_4$) m/z 325 ($MH^+$ for $^{80}Se$). Anal. Calcd. for $C_{16}H_{17}FOSe$: C,

59.44; H, 5.30. Found: C, 59.50; H, 5.29

## EXAMPLE 2

### Process for Preparing 3-(4 Methoxyphenyl)-2-Fluoro-1-Propene (I)

A solution of the compound of example 1 (1.0 g, 3 mmol) in carbon tetrachloride (100 ml) was cooled to -20 °C, and ozone was bubbled through the solution until a light blue color persisted. Diisopropylamine (0.6 g, 6 mmol) was added and the solution was heated at reflux for 16 hours, washed with ice cold dilute hydrochloric acid (2 times at 50 ml each), aqueous sodium carbonate (50 ml) and dried over magnesium sulfate. The solution was evaporated to an oil and purified by flash chromatography (hexane) to provide 415 mg (83%) of 3-(4-methoxyphenyl)-2-fluoro-1-propene as a colorless oil. Kugelrohr distillation of a small sample at 50 - 60 °C (0.05 mm) provided analytically pure 3-(4-methoxyphenyl)-2-fluoro-1-propene.

1H NMR (300 NHz, CDCl3) δ 3.43 (d, 2, J = 14.9 Hz), 3.80 (s, 3), 4.21 (ddt, 1, J = 49.4, 2.8, 0.9 Hz) 4.58 (dd, 1, J = 16.8, 2.7 Hz) 6.86 (d, 2, J= 8.7 Hz), 7.17 (d, 2, J = 8.7 Hz); $^{19}F$ NMR ($CDCl_3$) δ (vs. $CFCl_3$, 282 MHz) -95.0 (ddt, J = 49.1, 15.8, 15.2 Hz); MS Cl/$CH_4$) m/z 167 ($MH^+$). Anal. Calcd. for $C_{10}H_{11}FO$: C, 72.27; H, 6.67. Found C, 72.33; H, 6.78.

## EXAMPLE 3

### Process for Preparing 4-Allyl-1-(4-Methoxybenzyloxy)Benzene (II)

4-Allylphenol, (6.14 g, 0.046 moles), 4-methoxybenzyl chloride, (6.3 ml, 0.046 moles), potassium carbonate (8.3 g, 0.06 moles) and a catalytic amount of potassium iodide were mixed together in about 200 ml acetone, and heated to reflux, with stirring. The reaction was allowed to proceed overnight, then the mixture was cooled to room temperature and diluted with about 100 ml water. The product was extracted into ethyl acetate (3 times, 300 ml each time), and then dried over magnesium sulfate/potassium carbonate, filtered and concentrated <u>in vacuo</u> to yield the title compound as a light yellow solid, m.p. 59-61 °C.

## EXAMPLE 4

### Process for Preparing 3-(4-Methoxybenzyloxyphenyl) 2-Fluoro-1-(Phenylselenyl)Propane (III)

Phenylselenium chloride (1.81 g, 9.432 mmole) and 2.5 g (19.65 mmole) silver fluoride were each weighed into separate oven-dried, single necked flasks in a glove bag under nitrogen. Acetonitrile (15 ml) was added to each flask. Two grams of the compound of example 3 was dissolved in 15 ml acetonitrile and added to the flask containing silver fluoride. The phenylselenium chloride was added next, and the reaction was stirred at room temperature overnight. The reaction was filtered through a small amount of silica gel, eluted with chloroform and concentrated under reduced pressure to yield 2.94 g oil. Purification by flash chromatography (10% ether/hexane) produced 1.83 g white solid (yield of 64.3%).
Anal. Calcd. for $C_{23}H_{23}FO_2Se$: C, 64.33; H, 5.40. Found for $C_{23}H_{23}FO_2Se$: C, 64.42; H, 5.37.

## EXAMPLE 5

**Process for Preparing 3-[(4-Methoxybenzyl)-4-Oxyphenyl]-2-Fluoro-1-Propene**

In a manner similar to that described in example 2, 1.37 g (3.19 mmole) of the compound of example 4 was dissolved in 100 ml carbon tetrachloride, cooled to -20° C, and treated with ozone until a blue color persisted. The reaction was quenched with 323 mg (3.19 mmole) diisopropylamine and refluxed overnight. Purification by flash chromatography (20% ether/hexane) gave 387 mg (66.4% title compound based on recovered starting material.

Anal. Calcd. for $C_{17}H_{17}FO_2$: C, 74.97, H, 6.29. Found for $C_{17}H_{17}FO_2$: C, 74.50, 74.32; H, 6.10, 6.23. m.p. 77-78° C

## EXAMPLE 6

**Process for Preparing 3-(4-Hydroxyhenyl)-2-Fluoro-1-Propene**

3-[(4-Methoxybenzyl)-4-oxyphenyl]-2-fluoro-1-propene, i.e., the compound of example 5, (30 mg, 0.110 mmole) was treated with saturated methanolic hydrochloric acid and stirred at room temperature for one hour. The solution was then poured onto 50 ml cold saturated sodium chloride, diluted with 20 ml ether and the pH adjusted to pH 10 with cold 0.5 N sodium hydroxide. The mixture was washed 3 additional times with 15 ml portions of 0.5 N sodium hydroxide, and the combined aqueous layers were then washed with 50 ml ether. The aqueous layers were then acidified with cold 1N HCl to pH 1, solid NaCl was added and the product extracted with ether (3 x 50 ml), dried ($MgSO_4$), and concentrated under reduced pressure to give 8 mg clear oil (40%).

$^1H$ NMR (300 MHz, $COA_3$) δ 3.42 (d, 2, J = 15 Hz), 4.22 ddt, 1, J = 46.5, 2.1, 0.6 Hz), 4.61 (dd, 1, J = 16.8, 2.7 Hz), 6.79 (d, 2, J = 8.4 Hz), 7.12 d, 2, J = 8.7 Hz);

$^{19}F$ NMR ($COA_3$) (vs. $CFA_3$, 282 MHz) -95.05 (ddt, J = 6.64,49.1 15.2 Hz) MS (Cl/$CH_4$) m/z 153 (MH+)

HRMS Calcd. for $C_9H_9FO$: 152.0367. Found 152.0636.

## EXAMPLE 7

**Process for Preparing 3-Phenoxy-2-Fluoro-1-Propene (I)**

By substituting allylphenylether for 4-allylanisole of example 1, and by following the process set forth in examples 1 and 2, the title compound was made in 67% yield.

Anal. Calcd. for $C_9H_9FO$: C, 71.04; H, 5.96; Found: C, 71.11; H, 5.99.

In a like manner, by substituting the following compounds for 4-allylanisole (i.e., a compound of formula II) and by following the procedure set forth in examples 1 and 2, the following compounds can be made:

start with 3-phenyl-1-propene to yield 2-fluoro-3-phenyl-1-propene;
start with 2-cyclohexylethylene to yield 2-fluoro-2-cyclohexylethylene;
start with 2-cyclopentylethylene to yield 2-fluoro-2-cyclopentylethylene;
start with vinylnaphthylene to yield 2-(β-fluorovinyl) naphthylene;
start with 3-(2-thienyl)-1-propene to yield 2-fluoro-3-(2-thienyl)-1-propene;
start with 3-(3-thienyl)-1-propene to yield 2-fluoro-3-(3-thienyl)-1-propene;
start with 2-cyclododecayl ethylene to yield 2-fluoro-2-cyclododecayl ethylene;
start with 3-(2-tetrahydrofuranyl)-1-propene to yield 2-fluoro-3-(2-tetrahydrofuranyl)-1-propene;
start with 3-(4-pyridinonyl)-1-propene to yield 2-fluoro-3-(4-pyridinonyl)-1-propene;
start with 3-(2-thiazoyl)-1-propene to yield 2-fluoro-3-(2-thiazoyl)-1-propene;
start with 3-phenyl-1-butene to yield 2-fluoro-3-phenyl-1-butene;
start with 3-(2-thienyl)-1-butene to yield 2-fluoro-3-(2-thienyl)-1-butene;
start with 1,9-decadiene to yield 2,8-difluoro-1,9-decadiene;

start with 1-decene to yield 2-fluoro-1-decene;
start with 3,3-dimethyl-1-butene to yield 2-fluoro-3,3-dimethyl-1-butene;
start with 1-dodecene to yield 2-fluoro-1-dodecene;
start with 1-eicosene to yield 2-fluoro-1-eicosene;
start with 1-heptene to yield 2-fluoro-1-heptene;
start with 1-hexadecene to yield 2-fluoro-1-hexadecene;
start with 1-hexene to yield 2-fluoro-1-hexene;
start with 3-methyl-1-hexene to yield 2-fluoro-3-methyl-1-hexene;
start with methyl-1-pentene to yield 2-fluoro-3-methyl-1-pentene;
start with 4-methyl-2-pentene to yield 2-fluoro-4-methyl-2-pentene;
start with 1-nonene to yield 2-fluoro-1-nonene;
start with 1-octadecene to yield 2-fluoro-1-octadecene;
start with 1,7-octadiene to yield 2,6-difluoro-1,7-octadiene;
start with 1-octene to yield 2-fluoro-1-octene;
start with 1-pentadecene to yield 2-fluoro-1-pentadecene;
start with 1-tetradecene to yield 2-fluoro-1-tetradecene;
start with 1-tridecene to yield 2-fluoro-1-tridecene.

Also, by substituting the following starting materials for 4-allylphenol of example 3, and by follcwing the procedure set forth in examples 3 through 6, the following compounds can be made:

start with 3-methyl-4-(4-methoxybenzyloxy)allylbenzene to yield 3-(3-methyl-4-hydroxyphenyl)-2-fluoro-1-propene;

start with 3,5-dichloro-4-(4-methoxybenzyloxy)allylbenzene to yield 3-(3,5-dichloro-4-hydroxyphenyl)-2-fluoro-1-propene.

It should be apparent to one of ordinary skill in the art that changes and modifications can be made to this invention without departing from the spirit or scope of the invention as it is set forth herein.

## Claims

1. A process for preparing compounds of the formula I

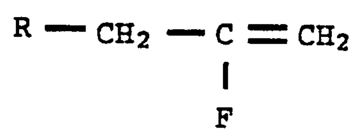

(I)

wherein R is a monovalent or bivalent cyclic group selected from the group consisting of optionally substituted $(C_3\text{-}C_{12})$monocyclic hydrocarbons: optionally substituted 5 and 6 membered aromatic heterocycles: $(C_3\text{-}C_{10})$heterocycles; and fused and bridged polycyclic groups; or R is monovalent or bivalent and is an optionally substituted $(C_1\text{-}C_{16})$alkyl; an optionally substituted $(C_1\text{-}C_{16})$-heteroalkyl; or a combination of a cyclic group and an alkyl or heteroalkyl group as described above; and wherein the optional substituents are 1 or more substituents selected from the group consisting of $C_1\text{-}C_{16}$)-alkyl, $(C_1\text{-}C_{16})$alkoxy, hydroxy, amine, protected sulfur and nitrogen derivatives, ketone, acetal, ketal, fluoro, chloro, and bromo which comprises:

a) forming a compound of the formula (III)

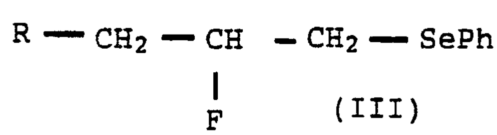

(III)

wherein SePh is phenylselinide,
by reacting phenylselenyl chloride or phenylselenyl bromide and silver fluoride with a compound of the formula (II)
$X\text{-}CH_2\text{-}CH=CH_2$ (II)
wherein X is R or a protecting group of R,
in an aprotic solvent;
b) treating the compound of formula III with ozone in a chlorinated solvent and at room temperature to -25° C; and
c) further reacting the compound of step b) with a non-nucleophilic amine at a temperature of 80° C to reflux.

2. A process for preparing compounds of the formula $I_a$

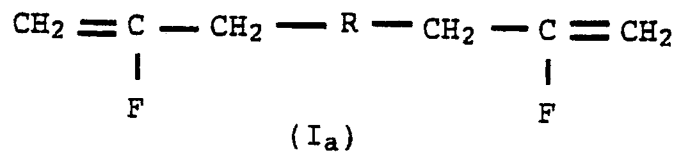

($I_a$)

wherein R is as defined in claim 1
which comprises:
a) forming a compound of the formula ($III_a$)

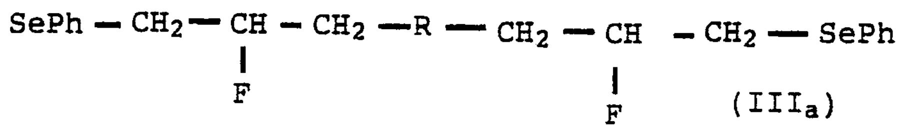

($III_a$)

wherein SePh is phenylselinide,
by reacting phenylselenyl chloride or phenylselenyl bromide and silver fluoride with a compound of the formula ($II_a$)
$H_2C=CH\text{-}CH_2\text{-}X\text{-}CH_2\text{-}CH=CH_2$ (IIa)
wherein X is R or a protecting group of R,
in an aprotic solvent;
b) treating the compound of formula IIIa with ozone in a chlorinated solvent and at room temperature to -25° C; and
c) further reacting the compound with a non-nucleophilic amine at a temperature of 80° C to reflux.

3. A process according to claims 1 or 2, wherein a compound of formula II or IIa is reacted with phenylselenyl chloride, and wherein the the aprotic solvent used is acetonitrile.

4. A process according to any of claims 1 to 3, wherein ozonalysis of step b) is carried out in carbon tetrachloride, and wherein the temperature is -15° C to -25° C.

5. A process according to any of claims 1 to. 4, wherein step c) is carried out at reflux, and wherein the amine utilized is diisopropylamine.

6. A process according to any of claims 1 to 5, wherein the ozone treatment continues until saturation.

7. A process according to claims 1 or 2, wherein a compound of formula II or IIa is reacted with phenylselenyl chloride and silver fluoride in acetonitrile to form a compound of formula III, which is reacted with ozone in carbon tetrachloride at -15° C to -25° C until saturated with ozone, and is then further reacted with diisopropylamine at reflux to produce a compound of formula I or Ia.

8. A process according to any of claims 1 and 3 to 7, wherein the product made thereby is 3-(4-methoxyphenyl)-2-fluoro-1-propene, 3-[(4-methoxybenzyl)-4-oxyphenyl]-2-fluoro-1-propene, 3-(4-hydroxyphenyl)-2-fluoro-1-propene, or 3-phenoxy-2-fluoro-1-propene.